## Europäisches Patentamt
## European Patent Office
## Office européen des brevets

(11) Veröffentlichungsnummer: **0 127 603**
**B1**

(12) **EUROPÄISCHE PATENTSCHRIFT**

(45) Veröffentlichungstag der Patentschrift:
04.01.89

(21) Anmeldenummer: 84890083.3

(22) Anmeldetag: 10.05.84

(51) Int. Cl.⁴: **A 61 K 35/16**, A 61 K 37/02

(54) **Verfahren zur Herstellung einer Faktor VIII(AHF)-hältigen Präparation.**

(30) Priorität: 20.05.83 AT 1858/83

(43) Veröffentlichungstag der Anmeldung:
05.12.84 Patentblatt 84/49

(45) Bekanntmachung des Hinweises auf die Patenterteilung:
04.01.89 Patentblatt 89/1

(84) Benannte Vertragsstaaten:
AT BE CH DE FR GB IT LI NL SE

(56) Entgegenhaltungen:
AT-A-369 263
DE-A-2 854 381
US-A-4 210 580

BIOLOGICAL ABSTRACTS, Band 63, Nr. 9, 1. Mai 1977, Seite 4815, Nr. 48885, Philadelphia, PA, US; A. BUKURESTLIEV et al.: "Production of a more potent factor VIII preparation", & FOLIO HAEMATOL (LEIPZIG) 103(5): 742-745, 1976 (recd. 1977)

(73) Patentinhaber: **IMMUNO Aktiengesellschaft für chemisch- medizinische Produkte, Industriestrasse 72, A-1220 Wien (AT)**

(72) Erfinder: **Linnau, Yendra, Dr., Lavendlweg 24, A-1224 Wien (AT)**
Erfinder: **Schwarz, Otto, Dr., Celtesgasse 5, A-1190 Wien (AT)**

(74) Vertreter: **Wolfram, Gustav, Dipl.- Ing., Schwindgasse 7 P.O. Box 205, A-1041 Wien (AT)**

**Beschreibung**

Die Erfindung betrifft ein Verfahren zur Herstellung einer Faktor VIII (AHF)-hältigen Präparation.

Es sind bereits Faktor VIII (AHF)-Konzentrate bekannt, die aus menschlichem oder tierischem Plasma hergestellt werden und im Vergleich zum nativen Plasma eine erhöhte Aktivität an Faktor VIII aufweisen. Bekannte Verfahren zur Herstellung von Faktor VIII-Konzentraten bedienen sich als Fraktionierungsmaßnahmen einer Behandlung des Plasmas mit Äthanol, mit Äther, mit Polyäthylenglykol und/oder Glycin. Bekannt ist auch die Kryopräzipitation des Plasmas nach Pool (1965, "The New England Journal of Medicine" 273, 1443) oder die Kryoäthanolpräzipitation des Plasmas nach Johnson (Congr. Int. Soc. Blood Transf., Sydney, Australia, Abstracts of Paper, Seite 1109 (1966)).

In der DE-A1-2 516 186 ist des weiteren ein Verfahren beschrieben, bei welchem ein aus Blutplasma gewonnenes Kryopräzipitat mazerisiert, das mazerisierte Produkt in einer Citrat-Glucose-Pufferlösung suspendiert, zentrifugiert und der so erhaltene Pufferextrakt auf einen pH-Wert im Bereich von 6,0 bis 6,8 eingestellt wird. Unter diesen Bedingungen erfolgt eine Fällung unerwünschter Verunreinigungen, worauf der den Faktor VIII enthaltende verbleibende Rückstand sterilisiert und lyophilisiert wird. Ein nach diesem Verfahren gewonnenes Faktor VIII-Produkt weist eine nur geringe spezifische Aktivität an Faktor VIII-Einheiten/mg Protein auf. Weiters ist auch der Anteil an Immunglobulin G (IgG), bezogen auf die Faktor VIII-Einheiten, unerwünscht hoch.

Ähnliche Verfahren sind auch in der AT-B-349 639 sowie in den US-A-4 170 639 und 4 104 266 beschrieben, wobei ebenfalls ausgehend von Plasma ein Kryopräzipitat gewonnen, dieses in einer Pufferlösung im neutralen pH-Bereich gelöst wird, wobei unerwünschte Proteine abgeschieden werden, der Überstand mit Aluminiumhydroxid behandelt wird, um den Prothrombinkomplex abzuscheiden, und dann die Faktor VIII enthaltende Lösung konzentriert und lyophilisiert wird. Auch bei diesen Verfahren ist die spezifische Aktivität an Faktor VIII-Einheiten/mg Protein unerwünscht gering. So beträgt die spezifische Aktivität bei der Arbeitsweise nach der US-A-4 104 266 nach den dortigen Angaben nur 0,5 bis 0,6 Einheiten/mg Protein.

Eine weitere Methode zur Herstellung von Faktor VIII-Konzentraten besteht in der Behandlung des Plasmas mit Adsorptionsmitteln, wie Florigel®, Bentonit, Ionenaustauschern und permeationschromatographischen Mitteln.

Die Erfindung bezweckt die Vermeidung der geschilderten Nachteile und Schwierigkeiten und stellt sich die Aufgabe, eine Faktor VIII (AHF)-hältige Präparation mit einer spezifischen Aktivität von mindestens 1,5 Einheiten Faktor VIII/mg Protein sowie mit einem Anteil an Immunglobulin G (IgG) von 15 bis 30 mg/1000 Einheiten Faktor VIII und einem Anteil an Fibrinogen von 20 bis 40 mg/100 Einheiten Faktor VIII zu schaffen, unter Verwendung der Methode der Fällung unerwünschter Proteine, insbesondere der Hauptmenge des Fibrinogens, bei einem pH-Wert etwa im Neutralbereich.

Die Erfindung, mit der diese Aufgabe gelöst wird, besteht bei einem Verfahren der vorstehend bezeichneten Art darin, daß die Fällung der unerwünschten Proteine in Anwesenheit von sulfatisiertem Polysaccharid bei einem pH-Wert von 6 bis 7 durchgeführt wird, worauf nach Verwerfen des Niederschlages der den Faktor VIII enthaltende Überstand mit einem Proteinfällungsmittel in Anwesenheit von Salzen bei einem pH-Wert von 6 bis 7 behandelt wird, wobei ein den Faktor VIII enthaltender Niederschlag erhalten, dieser gelöst und gegebenenfalls dem Endprodukt ein Antithrombin III-Heparin-bzw. Antithrombin III-Heparinoid-Komplex zugefügt wird.

Durch das erfindungsgemäße Verfahren wird eine Präparation mit einer hohen spezifischen Aktivität von beispielsweise 1,5 bis 4,0 Einheiten Faktor VIII/mg Protein und mit einem geringen Immunglobulin-Gehalt von etwa 15 bis 30 mg/1000 Einheiten Faktor VIII erhalten. Eine Präparation dieser Art zeigt eine sehr gute Löslichkeit nach der Lyophilisierung. Die Rekonstitutionsdauer beträgt nicht mehr als 0,5 bis 4 min, die Wirtschaftlichkeit des erfindungsgemäßen Verfahrens ist sehr gut, die Ausbeuten hoch.

Gemäß einer bevorzugten Ausführungsform werden als sulfatisiertes Polysaccharid Heparinoide, wie Mucopolysaccharidpolyschwefelsäureester, Pentosan-polysulfat, Dextransulfat verwendet.

Ein vorteilhaftes Verfahren gemäß der Erfindung ist gekennzeichnet durch die Kombination der folgenden Maßnahmen:

- daß ein Kryopräzipitat in einem Citrat-Heparinoid-Puffer gelöst, auf einen pH-Wert von 6,0 bis 6,4 gestellt und die Suspension auf 0 bis 25°C, vorzugsweise 4 bis 8°C gekühlt wird, wobei inerte Proteine gefällt werden,

- daß nach Verwerfen des Niederschlages die gereinigte, Faktor VIII enthaltende überständige Lösung durch Ausfällung mit einem Proteinfällungsmittel, wie Äthylalkohol, bei einem pH-Wert von 6,0 bis 7,0, in Anwesenheit von höchstens 1,45 Mol Glycin und/oder mindestens einer Ionenstärke von 0,15 aufkonzentriert wird,

- daß der Faktor VIII-hältige Niederschlag in einer Glycin-Citrat-NaCl-Pufferlösung in Anwesenheit eines Antithrombin III-Heparinoid- oder Antithrombin III-Heparin-Komplexes gelöst und in haltbare Form gebracht wird.

Eine Variante dieser Ausführungsform besteht darin, daß nach Zugabe des Proteinfällungsmittels und der Salze zu der den Faktor VIII enthaltenden überständigen Lösung die erhaltene Suspension eingefroren und bei einer Temperatur von 0 bis 4°C wieder aufgetaut wird, wobei der Überstand verworfen und der den Faktor VIII enthaltende Niederschlag in der Glycin-Citrat-NaCl-Pufferlösung in Anwesenheit eines Antithrombin III-Heparinoid- oder Antithrombin III-Heparin-Komplexes gelöst und in haltbare Form gebracht wird.

Zweckmäßig wird dem Endprodukt zu Stabilisierungszwecken Albumin zugefügt.

Unter der spezifischen Faktor VIII-Aktivität der erfindungsgemäß hergestellten Präparation wird das Verhältnis der Faktor VIII-Aktivität/mg Protein verstanden. Die Faktor VIII-Aktivität wird nach der sogenannten Zwei-Stufen-Methode, u. zw. nach D. E. G. Austen und I. L. Rhymes, "A Laboratory Manual of Blood Coagulation", Blackwell Scientific Publications, 1975, bestimmt. Die Proteinkonzentration kann nach der in der Literaturstelle A. G. Gornall, C. S. Bardawill und M. M. David, J. Biol. Chem. 177, 751 (1949) beschriebenen Methode bestimmt werden.

Eine Methode zur Bestimmung des Immunglobulins G (IgG), die für die erfindungsgemäßen Präparationen angewendet werden kann, ist in der Literatur, u. zw. Mancini G., Vaerman J. P., Carbonara A. O. und Heremans J. F., "A single-radial-diffusion method for the immunological quantitation of proteins", XI. Colloquium on protides of the biological fluids (1964) 370, Elsevier, Amsterdam, beschrieben.

Das Prinzip der Bestimmungsmethode besteht in der Reaktion zwischen Antigen (IgG) und Antikörper (Anti-IgG). Eine Immundiffusionsplatte aus Agarose (z. B. der Firma Immuno-Diagnostika) enthält das spezifische Antiserum (Anti-IgG).

Je 5 · 10⁻³ ml der Faktor VIII-hältigen Präparation und der Referenz-Standard-Präparation (mit bekanntem IgG-Gehalt, bezogen auf den WHO-Standard) werden in die Auftragslöcher aufgetragen. Nach mindestens 45 Stunden bei 20 bis 24°C Reaktionszeit werden die Durchmesser der kreisförmigen Präzipitation gemessen. Durch Vergleich mit dem definierten Immunglobulin-Referenz-Standard wird die Immunglobulin-Konzentration der Faktor VIII-hältigen Präparation bestimmt.

Die Bestimmung des Fibrinogens mittels Celluloseacetatmembran-Elektrophorese ist ebenfalls in der Literatur, u. zw. Michael D. Gebott, Beckman Microzone Electrophoresis Manual, Beckman Instruments, Inc. 1977, 015-083630-C beschrieben.

Gemäß der dort angegebenen Anleitung geht man in folgender Weise vor: Eine Celluloseacetat-Membran wird mit Veronal®/Veronalnatriumpuffer, pH-Wert 8,6 (Beckman B-2 Puffer), durchtränkt und äquilibriert. Mit Hilfe des Probenauftragsstempels werden 12,5 · 10⁻³ mg der Faktor VIII-hältigen Präparation auf die äquilibrierte Membran aufgetragen. Als Referenzsubstanz für die Einordnung der einzelnen Komponenten (Albumin, Alpha-Globulin, Beta-Globulin, Fibrinogen und Gamma-Globulin) wird humanes Plasma herangezogen. Für die Bestimmung der Wanderungsgeschwindigkeit der Komponenten wird als Standard humanes Albumin verwendet.

Sind alle Proben, Albumin und Plasma auf der Membran aufgetragen, wird die elektrophoretische Trennung in der Mikrozonen-Trennkammer (Beckman R-200) und mit dem Netzgerät (Beckman 4264) durchgeführt: elektrische Spannung 250 V, Stromstärke 3 bis 4 mA pro Membran, Zeitdauer 20 min.

Danach werden die Proteinbändchen mit Ponceau S angefärbt, die überschüssige Färbelösung wird mittels eines Gemisches von 1 Teil Essigsäure und 19 Teilen Methanol entfernt. Die Folie wird durch reines Methanol dehydratisiert und transparent gemacht durch Eintauchen in ein Gemisch von 1 Teil Essigsäure und 3 Teile Methanol. Anschließend wird die Folie auf einer Glasplatte getrocknet und densitometrisch (Beckman Densitometer R-112) ausgewertet; es druckt den relativen Fibrinogengehalt der Gesamtproteinkonzentration aus. Die absolute Fibrinogenmenge erhält man durch Multiplikation der relativen Fibrinogenkonzentration mit der Proteinkonzentration der Faktor VIII-hältigen Präparation.

Das erfindungsgemäße Verfahren wird durch die folgenden Beispiele näher erläutert:

**Beispiel 1:**

6660 ml gefrorenes frisches Plasma wurden bei 0°C bis +4°C aufgetaut. Das entstandene Kryopräzipitat wurde durch Zentrifugieren abgetrennt und in 700 ml Tri-natriumcitrat-Lösung, die 0,1 mg Dextransulfat (Pharmacia) pro ml und 30 Einheiten Aprotinin pro ml enthielt, gelöst. Der pH-Wert der Lösung wurde auf 6,3 und die Temperatur auf 4°C gestellt. Dabei entstand ein Niederschlag, der durch Zentrifugieren abgetrennt und verworfen wurde.

Durch Zugabe von 8 % Äthanol wurde die Faktor VIII-hältige Fraktion ausgefällt, wobei die Immunglobuline (IgG, IgA, IgM) zum großen Teil in Lösung gehalten wurden durch Zusatz von Aminosäuren, wie 10 % Glycin, oder durch Erhöhung der Ionenstärke mittels NaCl oder Natriumcitrat. Der abgetrennte Niederschlag, der die Faktor VIII-hältige Fraktion enthielt, wurde in einem Glycin-Citrat-NaCl-Puffer gelöst, der einen Antithrombin III-Heparin-Komplex mit einer Antithrombin-Konzentration von 0,05 E pro ml enthielt; sodann wurde die gelöste Präzipitation in Endbehälter abgefüllt und lyophilisiert.

Die Herstellung dieses Antithrombin III-Heparin-Komplexes wurde wie folgt durchgeführt:

Zu 1 l Plasma wurden 80 000 E Heparin zugesetzt und es wurde 30 min bei +4°C gerührt. Nach dem Einrühren von 1 g DEAE-Sephadex® A 50 wurde weitere 2 Stunden bei +4°C gerührt. Das beladene Gel wurde mittels Büchnertrichter abgetrennt und zur Entfernung von Begleitproteinen zweimal mit je 100 ml einer Phosphat- und Citrat-gepufferten isotonen Kochsalzlösung gewaschen.

Das beladene, gewaschene Gel wurde in 50 ml des oben genannten Puffers suspendiert und durch Zugabe von festem NaCl wurde eine Leitfähigkeit von 42 mS/cm eingestellt. Nach einstündigem Rühren bei +4°C wurde mittels Büchnertrichter abgetrennt und der Antithrombin III-Heparin-Komplex im Eluat gewonnen, wobei das Eluat gegen Saline dialysiert wurde.

Die Kennwerte des nach diesem Beispiel erhaltenen Produktes - ermittelt nach den oben wiedergegebenen

Literaturstellen - sind:

| Faktor VIII-Einheiten/mg Protein | 1,5 | |
|---|---|---|
| IgG/1000 Einheiten Faktor VIII | 17,0 | mg |
| Fibrinogengehalt/100 Einheiten Faktor VIII | 35 | mg |
| Rekonstitutionszeit | 4 | min |

**Beispiel 2:**

Die Präparation wurde in gleicher weise wie in Beispiel 1 bereitet, jedoch wurde statt Dextransulfat ein Mucopolysaccharidpolyschwefelsäureester verwendet. Die Kennwerte bei diesem Produkt waren die folgenden:

| Faktor VIII-Einheiten/mg Protein | 3,1 | |
|---|---|---|
| IgG/1000 Einheiten Faktor VIII | 15,6 | mg |
| Fibrinogengehalt/100 Einheiten Faktor VIII | 30 | mg |
| Rekonstitutionszeit | 1 | min |

**Beispiel 3:**

Die Präparation wurde in gleicher Weise wie in Beispiel 1 bereitet, jedoch wurde statt Dextransulfat ein Pentosan-polysulfat (SP 54) verwendet und der Auflösungspuffer enthielt einen Antithrombin III-Heparinoid-Komplex in einer Konzentration von 0,05 Einheiten/ml. Dieser Antithrombin III-Heparinoid-Komplex wurde in folgender Weise hergestellt:

Zu 1 l Plasma wurden 3 g Polyanion SP 54 zugesetzt und 30 min bei +4°C gerührt. Nach dem Einrühren von 2,5 g DEAE-Sephadex A 50 wurde weitere 2 Stunden bei +4°C gerührt. Das beladene Gel wurde mittels Büchnertrichter abgetrennt und zur Entfernung von Begleitproteinen zweimal mit je 200 ml einer Phosphat- und Citrat-gepufferten, isotonen Kochsalzlösung gewaschen. Das beladene, gewaschene Gel wurde in 100 ml des oben genannten Puffers suspendiert und durch Zugabe von festem Kochsalz wurde eine Leitfähigkeit von 60 mS/cm eingestellt. Nach einstündigem Rühren bei +4°C wurde mittels Büchnertrichter abgetrennt und der Antithrombin III-Heparinoid (SP 54)-Komplex im Eluat gewonnen, wobei das Eluat gegen Saline dialysiert wurde.

Die Kennwerte bei dem nach diesem Beispiel hergestellten Produkt waren die folgenden:

| Faktor VIII-Einheiten/mg Protein | 2,47 | |
|---|---|---|
| IgG/1000 Einheiten Faktor VIII | 27,0 | mg |
| Fibrinogengehalt/100 Einheiten Faktor VIII | 33 | mg |
| Rekonstitutionszeit | 1 | min |

**Beispiel 4:**

7000 ml gefrorenes frisches Plasma wurden bei 0 bis +4°C aufgetaut. Das entstandene Kryopräzipitat wurde abgetrennt und in 550 ml Tri-Natriumcitrat-Lösung, die Mucopolysaccharidpolyschwefelsäureester enthält, gelöst. Der pH-Wert der Lösung wurde auf 6,65 und die Temperatur auf 1°C gestellt. Dabei entstand ein Niederschlag, der abgetrennt und verworfen wurde. Die Faktor VIII-hältige Fraktion wurde durch Zugabe von 8 % Äthanol und 7,5 % Glycin zum Überstand ausgefällt. Die Suspension wurde tiefgefroren und bei 0 bis +4°C wieder aufgetaut.

Nach Abtrennung des Faktor VIII-hältigen Niederschlages wurde dieser gelöst, abgefüllt und lyophilisiert. Die Kennwerte bei diesem Produkt waren die folgenden:

| | |
|---|---|
| Faktor VIII-Einheiten/mg Protein | 1,66 |
| IgG/1000 Einheiten Faktor VIII | 22,0 mg |
| Fibrinogengehalt/100 Einheiten Faktor VIII | 40 mg |
| Rekonstitutionszeit | 3 min |

**Beispiel 5:**

5000 ml gefrorenes frisches Plasma wurden bei 0 bis +4°C aufgetaut. Das entstandene Kryopräzipitat wurde abgetrennt und in 350 ml Tri-Natriumcitrat-Lösung, die Pentosanpolysulfat enthielt, gelöst. Der pH-Wert der Lösung wurde auf 6,20 und die Temperatur auf 8°C gestellt. Dabei entstand ein Niederschlag, der abgetrennt und verworfen wurde. Die Faktor VIII-hältige Fraktion wurde durch Zugabe von 15 % Glycin zum Überstand ausgefällt. Die Suspension wurde tiefgefroren und bei 0 bis +4°C wieder aufgetaut.

Nach Abtrennung des Faktor VIII-hältigen Niederschlages wurde dieser gelöst, abgefüllt und lyophilisiert. Die Kennwerte bei diesem Produkt waren die folgenden:

| | |
|---|---|
| Faktor VIII-Einheiten/mg Protein | 3,80 |
| IgG/1000 Einheiten Faktor VIII | 20 mg |
| Fibrinogengehalt/100 Einheiten Faktor VIII | 21 mg |
| Rekonstitutionszeit | 2 min |

**Patentansprüche**

1. Verfahren zur Herstellung einer Faktor VIII (AHF)-hältigen Präparation, mit einer spezifischen Aktivität von mindestens 1,5 Einheiten Faktor VIII/mg Protein sowie mit einem Anteil an Immunglobulin G (IgG) von 15 bis 30 mg/1000 Einheiten Faktor VIII und einem Anteil an Fibrinogen von 20 bis 40 mg/100 Einheiten Faktor VIII, wobei eine Faktor VIII-hältige Plasmafraktion in einer Pufferlösung gelöst, die Lösung durch Fällung unerwünschter Proteine bei einem pH-Wert im Neutralbereich gereinigt, der Faktor VIII-hältige Rückstand konzentriert und das Konzentrat in haltbare Form gebracht wird, dadurch gekennzeichnet, daß die Fällung der unerwünschten Proteine in Anwesenheit von sulfatisiertem Polysaccharid bei einem pH-Wert von 6 bis 7 durchgeführt wird, worauf nach Verwerfen des Niederschlages der den Faktor VIII enthaltende Überstand mit einem Proteinfällungsmittel in Anwesenheit von Salzen bei einem pH-Wert von 6 bis 7 behandelt wird, wobei ein den Faktor VIII enthaltender Niederschlag erhalten, dieser gelöst und gegebenenfalls dem Endprodukt ein Antithrombin III-Heparin- bzw. Antithrombin III-Heparinoid-Komplex zugefügt wird.

2. Verfahren nach Anspruch 1, dadurch gekennzeichnet, daß als sulfatisiertes Polysaccharid Heparinoide, wie Mucopolysaccharidpolyschwefelsäureester, Pentosan-polysulfat, Dextransulfat verwendet werden.

3. Verfahren nach Anspruch 1, gekennzeichnet durch die Kombination der folgenden Maßnahmen:
- daß ein Kryopräzipitat in einem Citrat-Heparinoid-Puffer gelöst, auf einen pH-Wert von 6,0 bis 6,4 gesteilt und die Suspension auf 0 bis 25°C, vorzugsweise 4 bis 8°C gekühlt wird, wobei inerte Proteine gefällt werden,
- daß nach Verwerfen des Niederschlages die gereinigte, Faktor VIII enthaltende überständige Lösung durch Ausfällung mit einem Proteinfällungsmittel, wie Äthylalkohol, bei einem pH-Wert von 6,0 bis 7,0, in Anwesenheit von höchstens 1,45 Mol Glycin und/oder mindestens einer Ionenstärke von 0,15 aufkonzentriert wird,
- daß der Faktor VIII-hältige Niederschlag in einer Glycin-Citrat-NaCl-Pufferlösung in Anwesenheit eines Antithrombin III-Heparinoid- oder Antithrombin III-Heparin-Komplexes gelöst und in haltbare Form gebracht wird.

4. Verfahren nach Anspruch 3, dadurch gekennzeichnet, daß nach Zugabe des Proteinfällungsmittels und der Salze zu der den Faktor VIII enthaltenden überständigen Lösung die erhaltene Suspension eingefroren und bei einer Temperatur von 0 bis 4°C wieder aufgetaut wird, wobei der Überstand verworfen und der den Faktor VIII enthaltende Niederschlag in der Glycin-Citrat-NaCl-Pufferlösung in Anwesenheit eines Antithrombin III-Heparinoid- oder Antithrombin III-Heparin-Komplexes gelöst und in haltbare Form gebracht wird.

5. Verfahren nach den Ansprüchen 1 bis 4, dadurch gekennzeichnet, daß dem Endprodukt zu Stabilisierungszwecken Albumin zugefügt wird.

**Claims**

1. Method for producing a Factor VIII (AHF) containing preparation having a specific activity of at least 1.5 units of Factor VIII/mg protein as well as a portion of immunoglobulin G (IgG) of from 15 to 30 mg/1000 units of Factor VIII and a portion of fibrinogen of from 20 to 40 mg/100 units of Factor VIII, by dissolution of a Factor VII containing plasma fraction in a buffer solution, purification of the solution by precipitation of undesired proteins at a pH in the neutral range, concentration of the Factor VIII containing residue and processing of the concentrate into stable form, characterised in that the precipitation of undesired proteins is carried out in the presence of sulfated polysaccharide at a pH of from 6 to 7, whereupon, after discarding the precipitate, the Factor VIII containing supernatant is treated with a protein precipitating agent in the presence of salts at a pH of from 6 to 7 so as to obtain a Factor VIII containing precipitate, which Factor VIII containing precipitate is dissolved, and if desired, an antithrombin III-heparin complex or an antithrombin III-heparinoid complex is added to the final product.

2. Method according to claim 1, characterised in that heparinoids, such as mucopolysaccharide polysulfuric acid ester, pentosan polysulfate, dextran sulfate, are used as sulfated polysaccharide.

3. Method according to claim 1, characterised by the combination of the following measures:
- dissolving a cryoprecipitate in a citrate-heparinoid buffer, adjusting it to a pH of from 6.0 to 6.4, and cooling the suspension to 0 to 25°C, preferably 4 to 8°C, with inert proteins being precipitated thereby,
- after discarding the precipitate, concentrating the purified Factor VIII containing supernate solution by precipitation with a protein precipitating agent, such as ethyl alcohol, at a pH of from 6.0 to 7.0 in the presence of 1.45 mol glycine at most and/or an ionic strength of 0.15 at least,
- dissolving the Factor VIII containing precipitate in a glycine-citrate-NaCl buffer solution in the presence of an antithrombin III-heparinoid complex or an antithrombin III-heparin complex and processing it into stable form.

4. Method according to claim 3, characterised in that after addition of the protein precipitating agent and the salts to the Factor VIII containing supernate solution, the suspension obtained is frozen and re-thawed at a temperature of from 0 to 4°C, the supernatant being discarded and the Factor VIII containing precipitate being dissolved in the glycine-citrate-NaCl buffer solution in the presence of an antithrombin III-heparinoid complex or an antithrombin III-heparin complex and being processed into stable form.

5. Method according to claims 1 to 4, characterised in that albumin is added to the final product for stabilising purposes.

**Revendications**

1. Procédé de préparation d'une composition contenant le facteur VIII (AHF), avec une activité spécifique d'au moins 1,5 unités de facteur VIII/mg de protéine, une teneur en immunoglubuline G (IgG) de 15 à 30 mg/1000 unités de facteur VIII et une teneur en fibrinogène de 20 à 40 mg/100 unités de facteur VIII par dissolution d'une fraction de plasma contenant le facteur VIII dans une solution tampon, purification de la solution par précipitation des protéines indésirables à un pH voisin de la neutralité, concentration du résidu contenant le facteur VIII et transformation du concentré en une forme conservable, caractérisé en ce que la précipitation des protéines indésirables est effectué en présence d'un polysaccharide sulfaté à un pH de 6 à 7, le liquide surnageant contenant le facteur VIII, après rejet du précipité, est traité par un agent précipitant des protéines en présence de sels à un pH de 6 à 7, ce qui donneun précipité contenant le facteur VIII qu'on dissout après quoi, le cas échéant, on ajoute au produit final un complexe antithrombine III-héparine ou antithrombine III-héparinoïde.

2. Procédé selon la revendication 1, caractérisé en ce que l'on utilise en tant que polysaccharide sulfaté un héparinoïde tel qu'un ester polysulfurique de mucopolysaccharide, du polysulfate de pentosane, du sulfate de dextrane.

3. Procédé selon la revendication 1, caractérisé par la combinaison des stades opératoires suivants:
- on dissout un cryoprécipité dans un tampon citratehéparinoïde, on règle à un pH de 6,0 à 6,4 et on refroidit la suspension à une température de 0 à 25°C, de préférence de 4 à 8°C, ce qui provoque la précipitation de protéines inertes,
- après rejet du précipité, on concentre la solution surnageante purifiée contenant le facteur VIII par précipitation à l'aide d'un agent précipitant des protéines tel que l'alcool éthylique, à un pH de 6,0 à 7,0, en présence de 1,45 mole au maximum de glycine et/ou à une force ionique d'au moins 0,15,
- on dissout le précipité contenant le facteur VIII dans une solution tampon glycine-citrate-NaCl en présence d'un complexe antithrombine III-héparinoïde ou antithrombine III-héparine et on le met sous une forme conservable.

4. Procédé selon la revendication 3, caractérisé en ce que, après addition de l'agent précipitant des protéines et des sels à la solution surnageante contenant le facteur VIII, on congèle ta suspension obtenue et on la décongèle à une température de 0 à 4°C, on rejette le liquide surnageant et on dissout le précipité contenant le facteur VIII dans la solution tampon glycinecitrate-NaCl en présence d'un complexe antithrombine III-héparinoïde ou antithrombine III-héparine et on met le produit sous une forme apte à la conservation.

5. Procédé selon les revendications 1 à 4 caractérisé en ce que l'on stabilise le produit final en lui ajoutant de l'albumine.